# EUROPEAN PATENT SPECIFICATION

(11) **EP 4 221 608 B1**
(45) Date of publication and mention of the grant of the patent: **06.11.2024**
(21) Application number: 21790788.0
(22) Date of filing: 29.09.2021
(51) Int. Cl.: A61B 17/80, A61B 17/90, A61B 17/88

(54) **A DEVICE FOR FIXING TO A BONE IN A GROWTH PLATE AREA**
VORRICHTUNG ZUR BEFESTIGUNG AN EINEM KNOCHEN IN EINEM WACHSTUMSPLATTENBEREICH
DISPOSITIF DE FIXATION À UN OS DANS UNE ZONE DE PLAQUE DE CROISSANCE

(30) Priority: 29.09.2020 DK PA202070663
(43) Date of publication of application: 09.08.2023
(73) Proprietor: Region Nordjylland, 9220 Aalborg Øst (DK)
(72) Inventor: ABOOD, Ahmed Abdul-Hussein, 9220 Aalborg Øst (DK)
(74) Representative: Plougmann Vingtoft a/s
(86) International application number: PCT/EP2021/076803
(87) International publication number: WO 2022/069548

(56) References cited:
- EP-A1- 3 257 458
- US-A1- 2006 142 767
- US-A1- 2007 093 834
- US-A1- 2013 267 955
- US-A1- 2018 021 050

## Description

### FIELD OF THE INVENTION

The present invention relates to a device for fixing to a bone in a growth plate area. The device comprises a rigid body with two elongate openings having a longitudinal axis and a width. The longitudinal axis of the two elongate openings are offset from each other in a direction of the width of the elongate openings, and orientated in directions which preferably are at the most 5 degrees different from each other, such as being parallel. With such a device arranged on a bone by screws going through the elongate opening as disclosed herein, a rotation of the bone can be obtained by the growth of the bone.

### BACKGROUND OF THE INVENTION

Rotational deformities (maltorsion) in one or more legs of a human being may result in pain and discomfort, not only in the leg itself but also in the hips and/or back in addition to effect on gait. Maltorsion of the lower limb is currently treated by surgical osteotomy, de-rotation and fixation with a plate, intramedullary nail or external fixator. Such procedures are invasive and require a long hospital admission and considerable post-operative pain and stress in the treated children. This novel device presented herein intends to exploit the principle of guided growth, which is routinely applied in the treatment of coronal and sagittal deformities.

US 2007/093834A1 discloses a bone alignment implant includes a first bone fastener with a first bone engager that is adapted for fixation into the metaphyseal bone and a second bone fastener with a second bone engager that is adapted for fixation into the diaphyseal bone. A link connecting the two fasteners spans across the physis. Alternatively, the bone alignment implant is adapted for fixation into the diaphyseal sections of two adjoining vertebral bodies. These implants act as a flexible tethers between the metaphyseal and the diaphyseal sections of bone during bone growth. These implants are designed to adjust and deform during the bone realignment process. When placed on the convex side of the deformity, the implant allows the bone on the concave side of the deformity to grow. During the growth process the bone is then realigned. A similar procedure is used to correct torsional deformities.

### OBJECT OF THE INVENTION

It is the object of the present invention to provide a device for fixing to a bone in a growth plate area which, when in use, may at least mitigate the problems related with the known device.

It is the object of the present invention to provide an alternative to the prior art.

### SUMMARY OF THE INVENTION

Thus, the above described object is intended to be obtained in accordance with the invention by providing a device for fixing to a bone in a growth plate area according to claim 1.

Preferred embodiments are set forth in the dependent claims.

References to "embodiments" throughout the description which are not under the scope of the appended claims merely represent possible exemplary executions and are therefore not part of the present invention. In accordance with the present invention, a device for fixing to a bone in a growth plate area comprises a rigid body comprising two elongate openings each elongate opening having a longitudinal axis and a width; a first portion of the rigid body comprising one of the two elongate openings and having a first distal end, and a second portion of the rigid body comprising the other of the two elongate openings and having a second distal end, where distal refers relatively to a center of the device, the first portion and the second portion being connected to each other at a position proximal to the distal ends, wherein the first portion and the second portion both has a center line; wherein said device is configured such that a first screw having essentially the same or a smaller diameter as the width of the first elongated opening is configured to allow travelling, during longitudinal growth, along the longitudinal axis of the first elongated opening when said first screw is inserted in the first elongate opening, and a second screw having essentially the same or smaller diameter as the width of the second elongated opening is configured to allow travelling, during longitudinal growth, along the longitudinal axis of the second elongated opening when said second screw is inserted in the second elongate opening, wherein the longitudinal axis of the two elongate openings are offset from each other in a direction of the width of the elongate openings, and orientated in directions which are at the most 5 degrees different from each other, such as being parallel, and said center lines being offset from each other in a direction of the width of the elongate openings.

Preferred embodiments of a device according to invention, may further comprise a connecting portion connecting said first portion and said second portion. Preferably, the length of the connecting portion may be between 0.25 and 5.0 cm such as between 0.5 cm and 2.5 cm, preferably between 0.5 cm and 2.0 cm. The connecting portion may further comprise a plurality of through going openings, such as circular openings configured for k-wire fixation.

Preferred embodiments of the device according to the invention may comprise a connection axis typically passing through the middle of each end face of two elongate openings facing towards each other. In embodiments comprising said through going openings, the connecting axis may, alternatively, be defined as a line passing through the centers of at least two of said plurality of through going openings. The longitudinal axis of the first elongated opening typically forms a first opening angle with the connection axis, and the longitudinal axis of said second elongated opening typically forms a second opening angle with the connection axis. Preferably, the first opening angle and said second opening angle may be formed opposite each other relative connection axis and may each be between 20 and 150 degrees, preferably each be between 90 and 150 degrees. The first opening angle and said second opening angle may each be less than 155 degrees, preferably less than 135 degrees, preferably less than 120 degrees.

Preferably, the first and second elongated openings may each have a length at least two times their largest width. Preferably said first and second elongated openings may each have a length at least three times their largest width.

Preferably, the first and second elongated openings may each have a length between 0.5 cm and 10.0 cm, such as between 0.75 cm and 5.0 cm, preferably between 1.0 cm and 2.5 cm.

Preferably, the first and second elongated openings may each have a largest width between 2.0 mm and 6.0 mm, preferably a largest width between 3.0 mm and 6.0 mm, such as between 3.0 mm and 5.0 mm.

Preferably, the length of the device may be between 2.0 cm and 6.0 cm, preferably between 2.5 cm and 6.0 cm.

Preferably, the length of each of the first and second elongated openings may be at least one third of the length of the device.

Preferably, a combined area of said first and second elongated openings in the longitudinal plane of the device may comprise at least 50% of the combined area of said first and second portions in the longitudinal plane of the device.

Preferably, said first opening angle and second opening angle may be essentially equal.

Preferably, the longitudinal axis of said first elongated opening may mirror the longitudinal axis of said second elongated opening across an axis in the longitudinal plane of the device. Preferably, the longitudinal axes of said first elongated opening (3a) and second elongated opening (3b) may be essentially parallel in the longitudinal plane of the device.

Preferably, the shape of the first elongated opening and/or the second elongated opening may be stadium shaped. Alternatively or in combination, the shape of the first elongated opening and/or the second elongated opening may be a rectangle.

Preferably, wherein the shape of the first elongated opening and/or the second elongated opening may be curved in the longitudinal plane of the device. Preferably, said rigid body may be one of z-shaped, 8-shaped, hourglass shaped, double trapezoid shaped, or formed of a plurality of parallelograms.

Preferably, said device may be formed of one or more of the following materials: stainless steel (SS), cobalt base alloys, bioceramics, titanium alloys, pure titanium, composite materials, and polymers.

Preferably, the device may be formed in a curve typically along the length of the device from the first distal end to the second distal end, said curve may preferably have a radius of curvature between 1.5 cm and 7.0 cm.

Preferably, the first and second elongated openings may be at least partly extended in parallel in the longitudinal direction of the device, such that the elongate openings (3a, 3b) may at least partly overlap, such as at least 5%, such as at least 15%, such as at least 25% in a lateral direction of the device. Preferably the device may be made in a single piece and made of metal.

Further, the invention relates to a kit of parts for fixing the device according to the invention to a bone according to claim 15.

Preferably, the third screw and/or the fourth screw may have a head, a threaded part and preferably a non-threaded part between the head and the threaded part. Preferably, the kit of parts may comprise
- at least two devices according to the invention;
- a third screw typically having a diameter essentially the same or smaller than the width of the first elongated opening (3a) of a second device; and
- a fourth screw typically having a diameter essentially the same or smaller than the width of the second elongated opening of said second device.

The invention is useful in connection with a method of attaching a first device and second device according to the invention to a bone. The method may typically comprise:
- fixing the first device on the medial side of the bone, and fixing the second device on the lateral side of the bone, such that at least a portion of the first device and at least a portion of the second device are arranged over the growth plate, and such that the longitudinal axes of the first and second elongated openings of each of the first and second device are arranged oblique to the circumferential axis of the growth plate.

The first elongated opening of the first device is superior and has its longitudinal axis angled towards the anterior of the bone and the first elongated opening of the second device is superior and has its longitudinal axis angled towards the posterior of the bone and wherein the connecting part is placed aligned with the longitudinal axis of the bone in the sagital plane.

Preferably, the oblique angle of the first and second openings of the first device and/or second device to the circumferential axis of the growth plate may be selected to achieve a desired amount of rotation of the bone within a predetermined time frame. Preferably, the predetermined time frame is selected based on the expected growth rate of the bone.

Preferably, an angle of the longitudinal axis of the first elongated opening of the first device formed with the circumferential axis of the growth plate and an angle of the longitudinal axis of the first elongated opening of the second device formed with the circumferential axis of the growth plate may be essentially equal. Preferably, both devices may be placed aligned with the longitudinal axis in the sagittal plane with the proximal arm of the first plate facing anterior or posterior depending on desired rotation (inward or outward) and the device is placed with the proximal arm facing the opposite direction to allow rotation.

### BRIEF DESCRIPTION OF THE FIGURES

The present invention will now be described in more detail with regard to the accompanying figures. The figures show ways of implementing the present invention and are not to be construed as being limiting to other possible embodiments falling within the scope of the attached claim set.
Figure 1 illustrates two devices according to preferred embodiments of the invention; fig. 1A illustrates a first embodiment of a device in a three dimensional view (upper part seen from above and lower part is a side-view); fig. 1B illustrates a second embodiment of device in a line drawing; the difference between the embodiment of fig. 1A and fig. 1B is the positioning of the through going holes 5 as will be detailed below;
Figure 2 illustrates a device according to the invention when fixed to a bone of a human; upper part of fig. 2 illustrates the initial position of the device before rotation of the bone has been effectuated and lower part of fig. 2 illustrates the position of the device after rotation of the bone has been effectuated;
Figure 3 illustrates a kit-of-parts according to a preferred embodiment of the invention;
Figure 4 illustrates other embodiments of a device according to the present invention;
Figure 5A and 5B illustrate other embodiments of a device according to the present invention.

### DETAILED DESCRIPTION OF PREFERRED EMBODIMENTS

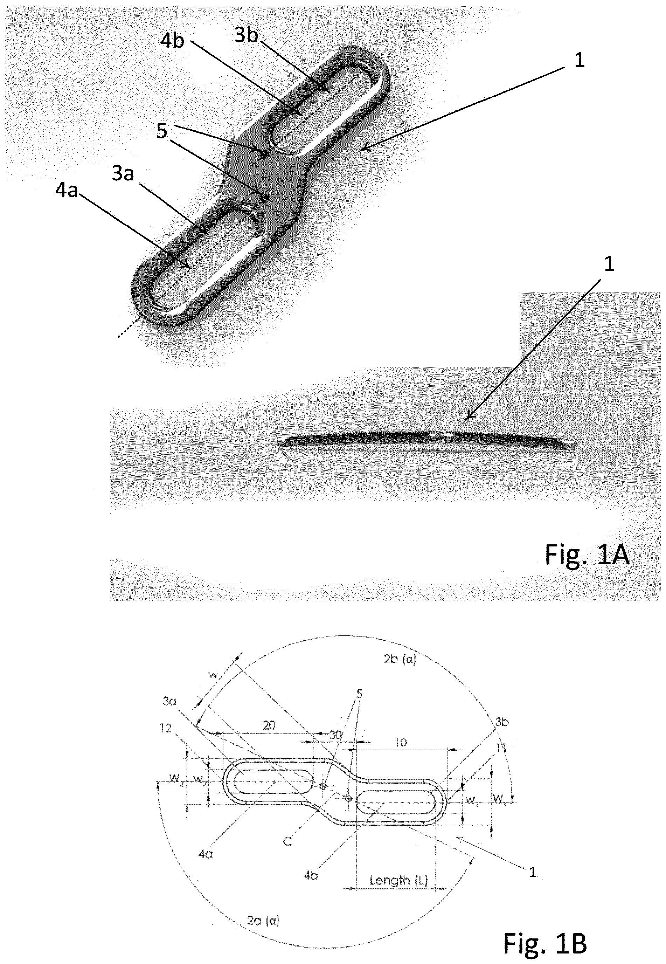

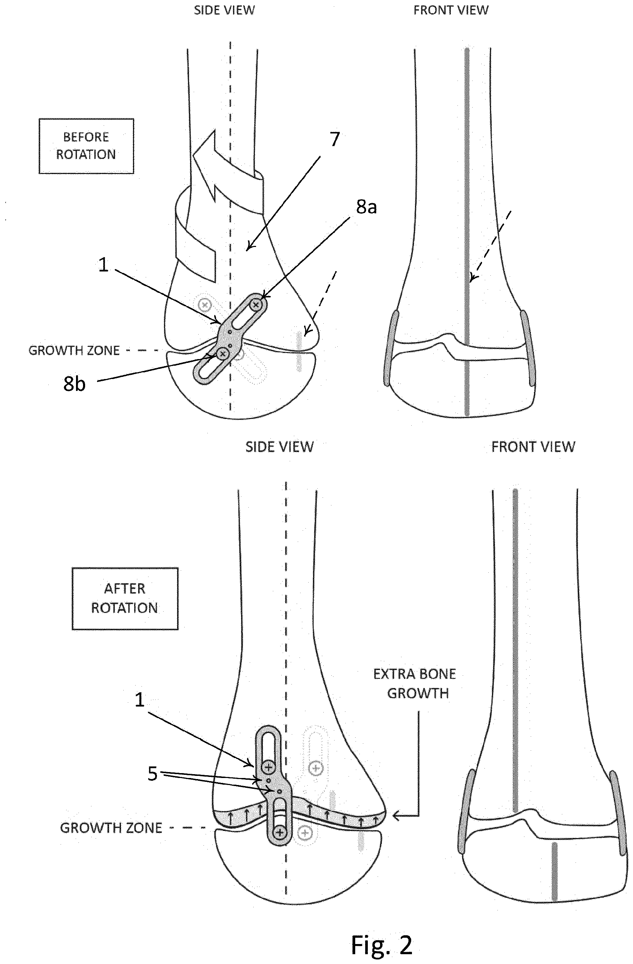

Reference is made to fig. 1A and 1B illustrating two devices according to preferred embodiments of the invention. As disclosed herein the device 1 is for fixing to a bone 7 in a growth plate area and this will be detailed with respect to fig. 2. The device of fig. 1A is in general as the one shown in fig. 1B except from the positioning of the through going openings 5. Thus, in the following a reference to fig. 1A and fig. 1B is made is made by a single reference to fig. 1 and with reference to the device 1, unless specific mentioned.

The shown device 1 has a rigid body comprising two elongate openings 3a, 3b. As shown these two elongate openings 3a, 3b each has two parallel sides connected by a section at the end of the elongate openings. Each elongate opening has a longitudinal axis 4a, 4b and a width w1, w2. The longitudinal axis of the two elongate openings 3a, 3b are offset from each other in a direction of the width of the elongate openings, and orientated in parallel directions.

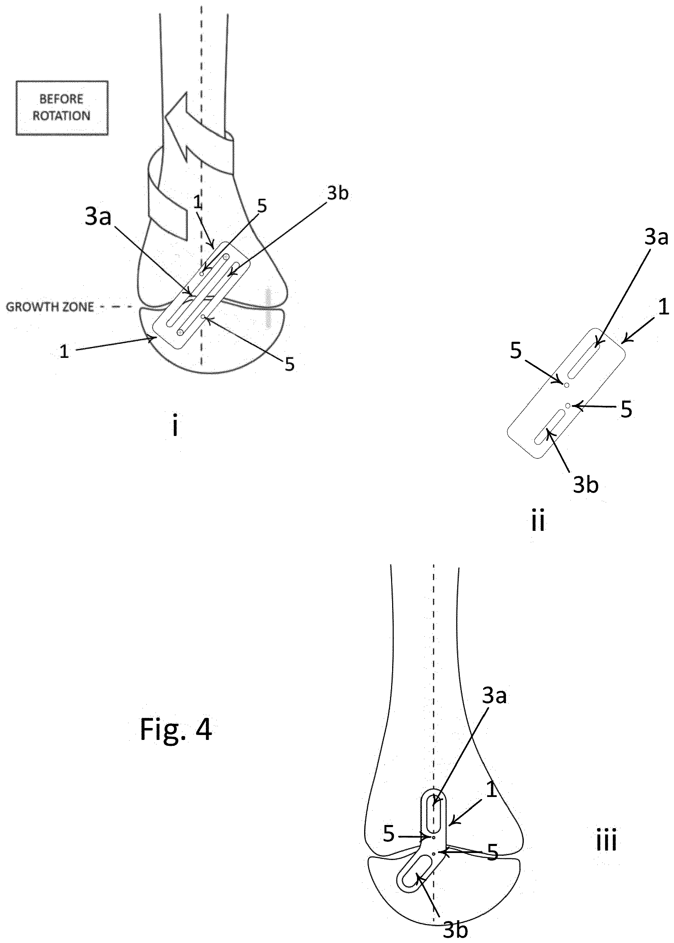

The longitudinal axis of the two elongate opening 3a, 3b, may be orientated in directions which are, preferably at the most 5 degrees different from each. One such example is shown in fig. 4 iii. Kindly note that the angle is shown exaggerated to make the angle more clearly identifiable.

It is noted that although the dimensions of the first and the second elongate openings 3a, 3b herein often are referred to as being different, e.g. the width w₁ and w₂ may be different, is it often preferred to shape the first and the second elongate openings to be similar, such as identical to each other.

Although not illustrated as such in fig. 1 the first portion 10 and the second portion 20 both has a center line. The center line is preferably defined as a line containing the geometrical mid-points in-between the outer edges of the first respectively second portion. In the illustrated embodiment, the longitudinal axis 4a and 4b are coinciding with the center lines due to the symmetrical arrangement of the elongate openings. However, in case one or both of the elongate openings are shifted toward one of the sides of the first and/or second portion, the center line will often depart from the longitudinal axis 4a and 4b. Thus, as seen, in preferred embodiments, the center line of the first portion and the center line of the second portion are offset from each other in a direction of the width of the elongate openings.

The device 1 is configured such that a first screw 8a (see fig. 2) having essentially the same or a smaller diameter as the width w₁ of the first elongated opening 3a is configured to allow travelling along the longitudinal axis 4a of the first elongated opening 3a when the first screw 8a is inserted in the first elongated opening 3a. Similarly, the device 1 is configured such that a second screw 8b (see fig. 2) having essentially the same or smaller diameter as the width w₂ of the second elongated opening 3b, is configured to allow travelling along the longitudinal axis 4b of the second elongated opening 3b when the second screw 8b is inserted in the second elongated opening 3b.

In typical preferred embodiments, the feature "configured to travel" is provided by the internal sides of the elongated openings 3a, 3b which, during use, contacts the sides of the screws 8a, 8b, which are mutually shaped so as to allow the internal sides of the elongated openings 3a, 3b to a slidingly contact to the side of the screws 3a, 3b.

Before detailing the device in further details, reference is made to fig. 2 illustrating the device when in use. Kindly note that the line shown on the bone and pointed at by dotted arrow are imaginary line used to show the rotation of the bone.

The upper part of fig. 2 shows that two devices 1 are placed aligned with the longitudinal axis in the sagittal plane and one device 1 pointing anterior and the other pointing posterior. The device on the hidden side of the bone is disclosed with phantom lines. The devices 1 are placed to span across the growth zone with the screws 8a, 8b screwed into the bone at locations above and below the growth zone.

The placement of the screws in relation to the plate will change during longitudinal growth as a result of the elongated openings. Since two devices 1 are used and they are placed by what might be labelled antisymmetric (as shown in fig. 2), the screws in the oblique elongated openings will create a rotational force acting on the bone throughout growth. This rotational force is provided by the travelling of first screw 8a and the second screw 8b which causes the two devices 1 to impart torque on the bone 7. The result of this rotational force is shown in the lower part of fig. 2 which shows the bone rotated.

With reference again to fig. 1, the device 1 will now be presented in further details. The device 1 may be disclosed as having a first portion 10 with a first distal end 11, and a second portion 20 with a second distal end 12. The labelling "distal" refers relatively to center of the device 1. As shown, the first portion 10 and the second portion 20 being connected to each other at a position proximal to the distal ends 11, 12. While the device 1 can be made with the first portion 10 and the second portion 20 as separated elements joined together, the device 1 is preferably made as a single element.

The device 1 of fig. 1 may also be disclosed as having a connecting portion 30. This connecting portion 30 connects the first portion 10 and the second portion 20, and may therefore be disclosed as an intermediate portion of the device. The connecting portion 30 of the device shown in fig. 1 extends in the region in-between the two elongate openings 4a, 4b. Also when the device is disclosed as having a connecting portion 30 it is preferred to make the device 1 as a single element, although the first portion 10, the second portion 20 and the connecting portion 30 may be formed as separate elements jointed to form a unified element. It is further noted that although the lengths of the first portion 10 and second portion 20 are illustrated as being equal, the length of the first portion 10 and the length of the second portion may be different. Similarly, the width and length of longitudinal openings may differ from each other.

In embodiments like the one of fig. 1, the connecting portion 30 can be disclosed as the portion of the device in-between two lines each being perpendicular to the longitudinal axes 4a or 4b of the two elongated openings which lines pass through the apex or end face of the longitudinal opening facing towards each other as illustrated in fig. 1B.

The length of the connecting portion 30 typically depends on a specific use, such as size of the bone to which it is to be applied and desired rotation. Typical sizes of the connecting portion 30 are between 0.25 and 5.0 cm such as between 0.5 cm and 2.5 cm.

The connection portion may also be disclosed as having a width W. The width W may be defined as the linear distance between two adjacent opposite outer edges of the connecting portion 30. This width W in combination with the thickness of device 1 is typically selected to provide sufficient rigidity to the device to avoid deformation during use and the smallest width of the connecting portion 30 is often less than half of the length of said first elongated opening 3a and less than half of the length of said second elongated opening 3b.

The device of fig. 1 can be disclosed as has having the plan view of a stretched z-shape with the largest width W₁ of said first portion 10, the largest width W₂ of said second portion 20 and the largest width W of said connecting portion 30 are essentially equal. In some embodiments, W may be larger or smaller than W₁ and/or larger than W₂.The widths W₁ and W₂ are measured in a linear direction perpendicular to the longitudinal axes 4a, 4b. These dimensions are not limited to devices having a plan view of a stretched z.

To allow fixation of the device to a bone, one or more through going openings 5 (such as circular) through which a k-wire (or similar) can be thread is provided in the device 1. This fixation is typically applied prior to inserting the screws 8a, 8b and is typically used to orientate the device 1 relatively to the bone and another device 1 arranged or to be arranged on the other adjacent side of the bone. The device 1 should be placed with one through going opening 5 (circular opening) proximal to the physis and one distal to it. In the embodiment of fig. 1, two of such through going openings 5 are provided in the connecting portion 30 in the form of two circular openings 5 configured for k-wire fixation.

By "k-wire" as used herein is typically meant Kirschner wires or technical equivalents thereto. Thus a k-wire typically refers to an elongate piece of metal, typically stainless steel, which has a smooth surface, being sterilized and sharpened at least at one end.

As perhaps most clear with reference to upper part of fig. 2, the openings 5 are positioned in the device so that they before rotation of the bone are aligned with the longitudinal direction of the bone with one opening 5 above and one opening below the growth zone. A device 1 is typically arranged on the bone by use of k-wires for temporarily fixation of the device 1. A k-wire is inserted into the bone trough the upper opening 5 and another through the lower opening 5 (or vice versa). Thereafter holes for the screws 8a, 8b (e.g. assisted by the light guide 15 - see below) are drilled and the screws screwed in the hole. The k-wires are then removed.

The through going openings 5, such as the circular openings, typically each has a diameter less than 1.7 mm, such as less than 1.6 mm, preferably less than 1.5 mm. The dimension, such as the diameter of the k-wire is typically chosen so that when introduced into the openings 5, a snug fit between the openings and the k-wire is produced.

As illustrated in fig. 1A, the through going openings 5 may each be placed with its geometrical centre on the longitudinal axis 4a, 4b, of the elongate opening 3a, 3b being closest to the through going opening, that is, with reference to fig. 1A, the opening 5 shown to the left is placed on the longitudinal axis 4a and the opening 5 shown to the right is placed on the longitudinal axis 4b.

The embodiments illustrated in fig. 1B is as the embodiment of fig. 1A except that the through going openings 5 are placed differently. Further, the embodiments of fig. 1A and fig. 1B may depending on the viewing angle be seen as being mirrored configurations with respect to the position of the elongate openings 3a, 3b. In fig. 1B the through going openings are placed offset from the longitudinal axis 4a 4b and in the particular shown embodiments, the through going openings 5 are placed on the connection axis C (see below as to the connection axis see).

The device may in some embodiments made in a single piece, and preferably made of metal, such as stainless steel, such as copper, such as titanium, such as aluminum, such as magnesium, such as cobalt chrome.

The device as shown in fig. 1 may be disclosed as having a connection axis C. Such a connection axis pass through the middle of each end face of two elongate openings 3a, 3b facing towards each other. By use of the connection axis C, it can be seen that the longitudinal axis 4a of the first elongated opening 3a forms a first opening angle 2a with the connection axis C, and that the longitudinal axis 4b of said second elongated opening 3b forms a second opening angle 2b with the connection axis C. In embodiments where the longitudinal axes 4a, 4b proceed in parallel, the two angles are equal. However, it is within the reach of the present invention to provide the device with different angles for 2a and 2b. The first opening angle 2a and said second opening angle 2b which are formed opposite each other relative to the connection axis C and may each be between 90 and 155 degrees. In particular embodiments, the angles 2a and 2b may be substantially 135 degrees, 120 degrees or 105 degrees.

In the embodiment shown in fig. 1, the shape of the first and the second elongate opening 3a and 3b is each stadium shaped in outline. By stadium shaped is referred to a geometrical shape constructed of a rectangle with semicircles at a pair of opposite ends. As can be seen in fig. 1A (upper part), the edges of the elongate openings 3a, 3b are rounded both in the plane visible in fig. 1A and on the other side (not visible). Such rounding is only preferred. By rounding of the edges, the friction between the screws 8a, 8b and point where the screws contact the elongate openings 3a, 3b can be reduced which may be beneficial e.g. to provide a smooth rotation of the bone.

While the semicircles of the stadium shape may be better adapted to accommodate the screws 8a, 8b when positioned at one of the ends of the elongate openings 3a, 3b, the device can function as desired when the shape of the first elongated opening 3a and the second elongated opening 3b is a rectangular in outline.

As the device is to be arranged on a bone structure not necessarily forming a flat surface, it is often advantageous to curve the device 1 to conform to, at least to some extent, the shape of bone. In many situations, the device is to be arranged at a joint, which in broad terms may be disclosed as curved. To take into account such curved bones (or for other reasons) the shape of the first elongated opening 3a and the second elongated opening 3b is curved in the longitudinal plane of the device 1. This can be seen in fig. 1A (lower part, side-view) where the device is lying on a flat surface. As can be realized from fig. 1A (side-view), a clearance is present between the flat surface and the underside of the device all the way between the points where the device rest on the surface, which points are the ends of the device. The device may in other embodiments be double-curved so that a curvature in directions perpendicular first one. The first portion and the second portion may have different or similar such as same curvatures.

For the embodiments shown in fig. 1 the device 1 is formed in a curve along the length of the device 1 from the first distal end 11 to the second distal end 12, said curve having a radius of curvature between 1.5 cm and 7.0 cm.

Since the device 1 is to be applied to a bone, the device should be made from a material, which is not instantaneously biologically rejected. Typical and preferred materials from which the device is made may be one or more of the following materials: stainless steel (SS), cobalt base alloys, bioceramics, titanium alloys, pure titanium, composite materials, and polymers.

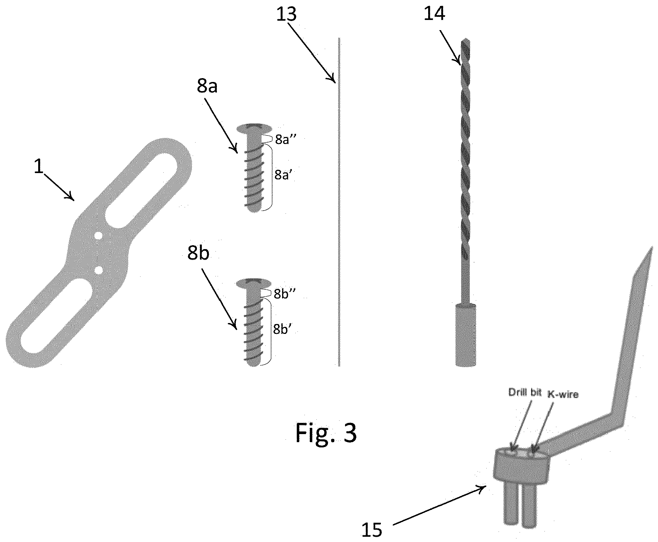

Reference is now made to fig. 3 illustrating a kit of parts for fixing a device 1 according to the invention to a bone 7. The kit of parts is shown as comprising one device 1, one first screw 8a having a diameter essentially the same or smaller than the width w1 of the first elongated opening 3a and one a second screw 8b having a diameter essentially the same or smaller than the width w2 of the second elongated opening 3b.

As shown in fig. 3, the screws 8a, 8b each has a threaded part 8a' and 8b' as well as a non-threaded part 8a" and 8b". The non-threaded part of each screw is positioned between the threaded part and the head. The purpose of having a non-threaded part is to reduce friction and/or scoring between the side of the elongate openings 3a, 3b and the screw, and the non-threaded parts are typically Machined, e.g. polished, to have a smooth surface. The longitudinal extension of the non-threaded parts are selected so that the thickness of the device can be accommodated in the region between the thread and the head of the screw. In embodiments, where the elongate openings have rounded sides (as disclosed in connection with fig. 1), the head of the screws and/or the non-threaded parts can be shaped to mimic such rounded sides.

The diameter as referred to above is typically the outermost diameter of the thread as this allows for screwing the screw into the bone by the screw going through the elongate opening and into the bone.

The screws are illustrated as screws with a fixed head including a notch for turning the screw. However, the invention is not limited to screws with a fixed head, since pin bolts may be used instead either as one or both of the screws 8a, 8b. In such embodiments, the pin bolts can be attached to the bone, where after the device 1, can be placed on the bolt and the bolt can be screwed on the top of the pin bolt. By this, the k-wire and the opening 5 can be omitted.

The kit of part may advantageously also comprise a k-wire 13 and a drill bit 14. A light guide 15 may also be included in the kit. The light guide 15 is used during application of the device 1 and comprising a first orifice configured to receive said drill bit and a second orifice configured to receive said k-wire. When the device is placed as desired by use of the k-wire, the light guide is arranged on the k-wire (which protrude from the bone and device 1) with the k-wire extending into the orifice configured to receive the k-wire. The leg of the light guide with the orifice configured to receive the drill bit is arranged in one of the elongated openings 3a, 3b and a hole for receiving a screw is drilled. The drilling direction is known by observing the direction of the k-wire.

In use two of such kit of parts are used for each bone and it may be beneficial to combine such two kits into a single kit of parts.

The kit of parts are typically sterilized as ordinarily done for equipment and parts for application to bones and the kit of parts are typically packed in a fluid tight sealed container, such as a pouch, preventing contaminations. Further a desiccant may be included in the container to absorb moisture that may have been trapped in the container before sealing.

Reference is made to fig. 4 illustrating three alternative embodiments of a device according to the present invention. These three embodiments are shown as fig. 4i, fig. 4ii and fig. 4iii respectively. Dimensions of the device as well as the shape of the elongate openings 3a, 3b can be as disclosed herein in relations to the e.g. the embodiment shown fig. 1.

The device 1 is in fig. 4i illustrated in a position of a bone, similarly to the illustration presented in fig. 2. The device 1 is formed by a rectangular rigid body having rounded outer corners to avoid sharp edges. The two elongate openings 3a, 3b extend in parallel in the longitudinal direction of the device 1. The length and the width of the elongate openings 3a, 3b are equal to each other, and the length of the elongate openings 3a, 3b are chosen so that the openings extend over a major part of the length of the rigid body. The elongate openings 3a, 3b are stadium shaped similarly to the openings described in connection with fig. 1.

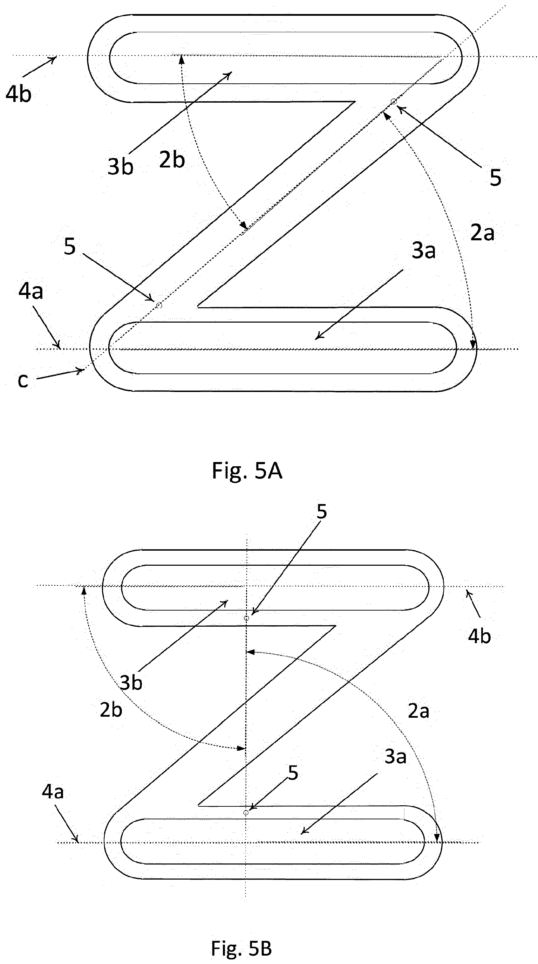

The elongate openings 3a, 3b may at least partly be extended in parallel in the longitudinal direction of the device 1, such that the elongate openings 3a, 3b at least partly overlap, such as at least 5%, such as at least 15%, such as at least 25% in a lateral direction of the device 1. The lateral direction of the device being the direction in which the width of the device is measured. By "overlap" is typically meant a configuration as illustrated in fig. 5A and fig. 5b. As can be seen in these figures, the elongate openings 3a, 3b, when seen in a side view overlaps which also may be referred to as the projections of the elongate openings 3a, 3b onto a plane being extending out from device and comprising one of the lines 4a or 4b are overlapping.

Fig. 5A and fig. 5B also illustrates an alternative definition of the first and the second opening angle. In embodiments comprising the through going opening 5, the connecting axis C may be defined as a line passing through the centers of both through going opening as illustrated in fig. 5A and 5B. The first and the second opening angle 2a, 2b may be defined relatively to that line C as also illustrated in fig. 5A and 5B. The embodiments shown in fig. 5A and 5B respectively differs from each other by the positioning of the through going openings 5.

The embodiment shown in fig. 4ii differs from the embodiment shown in fig. 1 by the rigid body being rectangular shaped with rounded outer corners.

The embodiment shown fig. 4iii differs from the embodiment shown in fig. 4ii by the two elongate openings 3a, 3b are arranged relatively to each other with longitudinal axis of the two elongate opening 3a, 3b, orientated in directions which are, preferably at the most 5 degrees different from each. One such example is shown in fig. 4 iii. Kindly note that the angle is shown exaggerated to make the angle more clearly identifiable.

The embodiments shown in fig. 4 are also shown with the optional openings 5 for the k-wire.

Devices 1 according to the invention may typically be attached to a bone in the following manner. As disclosed in connection with fig. 2, two devices 1 are typically applied and a preferred procedure to attached these two devices preferably comprises fixing the first device 1 on the medial side of the bone 7, and fixing the second device on the lateral side of the bone 7. The sequence of applying the first and the second device 1 can be selected arbitrarily, that is the labelling first and second does not indicate a mandatory sequence of fixing the devices 1. The devices are fixed such that at least a portion of the first device and at least a portion of the second device are arranged over the growth plate 6 and such that the longitudinal axes of the first and second elongated openings 3a, 3b of each of the first and second device are arranged oblique to the circumferential axis of the growth plate 6.

Further, the first elongated opening 3a of the first device is placed superior and has its longitudinal axis angled towards the anterior of the bone 7 and the first elongated opening 3a of the second device is superior and has its longitudinal axis angled towards the posterior of the bone 7 and wherein the connecting part is placed aligned with the longitudinal axis of the bone in the sagittal plane. It is noted that the orientation of the devices 1 determines whether the bone is rotated clock-wise or counter clock-wise. Thus, in another configuration, the devices could be mirrored, thus the superior elongated opening must face posterior aspect of the bone in the first device and anterior aspect in the second device to achieve reversed rotation.

As illustrated in fig. 2, the rotation of the bone 7 stops when the longitudinal axes 4a, 4b of the elongate openings 3a, 3b both are vertical or aligned with the growth direction of the bone. Thus, one can estimate a degree of rotation based on the before rotation position as there is relation between length growth and rotation due to the geometrical constraints imposed by the device 1.

This predetermined time frame is typically selected based on the expected growth rate of the bone 7.

Further, the two devices 1 are typically arranged so that that an angle of the longitudinal axis of the first elongated opening 3a of the first device 1 formed with the circumferential axis of the growth plate 6, and an angle of the longitudinal axis of the first elongated opening 3a of the second device formed with the circumferential axis of the growth plate 6 are essentially equal. Both devices are placed aligned with the longitudinal axis in the sagittal plane with the proximal arm of the first plate facing anterior or posterior depending on desired rotation (inward or outward) and the device is placed with the proximal arm facing the opposite direction to allow rotation.

Although the present invention has been described in connection with the specified embodiments, it should not be construed as being in any way limited to the presented examples. The scope of the present invention is set out by the accompanying claim set.

### List of reference symbols used

- 1: Device
- 2a: First angle
- 2b: Second angle
- 3a: First elongated opening
- 3b: Second elongated opening
- 4a: Longitudinal axis (of first elongated opening)
- 4b: Longitudinal axis (of second elongated opening)
- 5: Circular opening
- 6: Growth plate
- 7: Bone
- 8a: First screw
- 8b: Second screw
- 10: First portion
- 11: First distal end
- 12: Second distal end
- 13: K-wire
- 14: Drill bit
- 15: Light guide
- 20: Second portion
- 30: Connecting portion

## Claims

1. A device (1) for fixing to a bone (7) in a growth plate area, said device (1) comprising:
• a rigid body comprising two elongate openings (3a, 3b) each elongate opening having a longitudinal axis (4a, 4b) and a width (w1, w2);
• a first portion (10) of the rigid body comprising one of the two elongate openings and having a first distal end (11), and a second portion (20) of the rigid body comprising the other of the two elongate openings and having a second distal end (12), where distal refers relatively to a center of the device (1), the first portion (10) and the second portion (20) being connected to each other at a position proximal to the distal ends (11, 12), wherein the first portion (10) and the second portion (20) both has a center line;
wherein said device (1) is configured such that
• a first screw (8a) having essentially the same or a smaller diameter as the width (wi) of the first elongated opening (3a) is configured to allow travelling, during longitudinal growth, along the longitudinal axis (4a) of the first elongated opening (3a) when said first screw (8a) is inserted in the first elongate opening (3a), and
• a second screw (8b) having essentially the same or smaller diameter as the width (w₂) of the second elongated opening (3b) is configured to allow travelling, during longitudinal growth, along the longitudinal axis (4b) of the second elongated opening (3b) when said second screw (8b) is inserted in the second elongate opening (3b),
**characterized in that**
• the longitudinal axis of the two elongate openings (3a, 3b) are
∘ offset from each other in a direction of the width of the elongate openings, and
∘ orientated in directions which are at the most 5 degrees different from each other, such as being parallel, and
• said center lines being offset from each other in a direction of the width of the elongate openings (3a, 3b).

2. A device (1) according to claim 1, further comprising a connecting portion (30) connecting said first portion (10) and said second portion (20).

3. A device (1) according to claim 2, wherein the length of the connecting portion (30) is between is between 0.25 and 5.0 cm, such as between 0.5 cm and 2.5 cm, preferably between 0.5 cm and 2.0 cm.

4. A device (1) according to any of the claims 2 or 3, wherein the connecting portion (30) further comprises a plurality of through going openings, such as circular openings (5) configured for k-wire fixation.

5. A device according to claim 4, wherein the through going openings (5) each are placed with its geometrical centre on the longitudinal axis (4a, 4b) of the elongate opening (3a, 3b) being closest to the through going opening, or wherein the through openings (5) are placed offset from the longitudinal axis (4a, 4b).

6. A device according to any of the preceding claims, wherein the device has a connection axis (C) passing through the middle of each end face of two elongate openings (3a, 3b) facing towards each other or a connection axis (C) passing through the centers of at least two of said plurality of through going openings (5) defined in claim 4 and 5, and wherein the longitudinal axis (4a) of the first elongated opening (3a) forming a first opening angle (2a) with the connection axis (C), and the longitudinal axis (4b) of said second elongated opening (3b) forming a second opening angle (2b) with the connection axis (C), wherein said first opening angle (2a) and said second opening angle (2b) are formed opposite each other relative connection axis (C) and are each between 20 and 150 degrees, preferably each between 90 and 150 degrees.

7. A device (1) according to claim 6, wherein said first opening angle (2a) and said second opening angle (2b) are each less than 155 degrees, preferably less than 135 degrees, preferably less than 120 degrees.

8. A device (1) according to any of the previous claims, wherein the longitudinal axis of said first elongated opening (3a) mirrors the longitudinal axis of said second elongated opening (3b) across an axis in the longitudinal plane of the device (1).

9. A device (1) according to claim 8, wherein the longitudinal axes of said first elongated opening (3a) and second elongated opening (3b) are essentially parallel in the longitudinal plane of the device (1).

10. A device (1) according to any of the previous claims, wherein the shape of the first elongated opening (3a) and/or the second elongated opening (3b) is stadium shaped.

11. A device (1) according to any of the previous claims, wherein the shape of the first elongated opening (3a) and/or the second elongated opening (3b) is curved in the longitudinal plane of the device (1).

12. A device (1) according to any of the previous claims, wherein said rigid body is one of z-shaped, 8-shaped, hourglass shaped, double trapezoid shaped, or formed of a plurality of parallelograms.

13. A device (1) according to any of the previous claims, wherein the device (1) is formed in a curve along the length of the device (1) from the first distal end (11) to the second distal end (12), said curve preferably having a radius of curvature between 1.5 cm and 7.0 cm.

14. A device (1) according to any of the preceding claims, wherein the first and second elongated openings (3a, 3b) is at least partly extended in parallel in the longitudinal direction of the device (1), such that the elongate openings (3a, 3b) at least partly overlap, such as at least 5%, such as at least 15%, such as at least 25% in a lateral direction of the device (1).

15. A kit of parts for fixing the device (1) according to any of the preceding claims to a bone (7), comprising:
• at least one device (1) according to any of the preceding claims;
• a first screw (8a) having a diameter essentially the same or smaller than the width (wi) of the first elongated opening (3a);
• a second screw (8b) having a diameter essentially the same or smaller than the width (w₂) of the second elongated opening (3b);
• preferably, the first screw and/or the second screw a head, a threaded part and a non-threaded part between the head and the threaded part;
the kit for fixing the device preferably further comprising
• a k-wire (13);
• a drill bit (14); and
• a light guide (15) comprising a first orifice configured to receive said drill bit and a second orifice configured to receive said k-wire.

## Patentansprüche

1. Vorrichtung (1) zur Befestigung an einem Knochen (7) in einem Wachstumsplattenbereich, wobei die Vorrichtung (1) umfasst:
• einen starren Körper, der zwei längliche Öffnungen (3a, 3b) umfasst, wobei jede längliche Öffnung eine Längsachse (4a, 4b) und eine Breite (w1, w2) aufweist,
• einen ersten Abschnitt (10) des starren Körpers, der eine der zwei länglichen Öffnungen umfasst und ein erstes distales Ende (11) aufweist, und einen zweiten Abschnitt (20) des starren Körpers, der die andere der zwei länglichen Öffnungen umfasst und ein zweites distales Ende (12) aufweist, wobei sich distal relativ zu einem Zentrum der Vorrichtung (1) bezieht, wobei der erste Abschnitt (10) und der zweite Abschnitt (20) miteinander an einer Position proximal zu den distalen Enden (11, 12) verbunden sind, wobei der erste Abschnitt (10) und der zweite Abschnitt (20) beide eine Mittellinie aufweisen;
wobei die Vorrichtung (1) so konfiguriert ist, dass
• eine erste Schraube (8a), die im Wesentlichen den gleichen oder einen kleineren Durchmesser als die Breite (wi) der ersten länglichen Öffnung (3a) aufweist, so konfiguriert ist, dass sie während eines Längswachstums eine Bewegung entlang der Längsachse (4a) der ersten länglichen Öffnung (3a) ermöglicht, wenn die erste Schraube (8a) in die erste längliche Öffnung (3a) eingeführt ist, und
• eine zweite Schraube (8b), die im Wesentlichen den gleichen oder einen kleineren Durchmesser als die Breite (w₂) der zweiten länglichen Öffnung (3b) aufweist, so konfiguriert ist, dass sie während des Längswachstums eine Bewegung entlang der Längsachse (4b) der zweiten länglichen Öffnung (3b) ermöglicht, wenn die zweite Schraube (8b) in die zweite längliche Öffnung (3b) eingeführt ist,
**dadurch gekennzeichnet, dass**
• die Längsachse der beiden länglichen Öffnungen (3a, 3b)
∘ in einer Richtung der Breite der länglichen Öffnungen gegeneinander versetzt sind, und
∘ in Richtungen ausgerichtet sind, die sich um höchstens 5 Grad voneinander unterscheiden, wie beispielsweise parallel sind, und
• wobei die Mittellinien in einer Richtung der Breite der länglichen Öffnungen (3a, 3b) gegeneinander versetzt sind.

2. Vorrichtung (1) nach Anspruch 1, weiter umfassend einen Verbindungsabschnitt (30), der den ersten Abschnitt (10) und den zweiten Abschnitt (20) verbindet.

3. Vorrichtung (1) nach Anspruch 2, wobei die Länge des Verbindungsabschnitts (30) zwischen 0,25 und 5,0 cm, wie beispielsweise zwischen 0,5 cm und 2,5 cm, vorzugsweise zwischen 0,5 cm und 2,0 cm liegt.

4. Vorrichtung (1) nach einem der Ansprüche 2 oder 3, wobei der Verbindungsabschnitt (30) weiter eine Vielzahl von Durchgangsöffnungen, wie beispielsweise kreisförmige Öffnungen (5), aufweist, die für eine K-Draht-Befestigung konfiguriert sind.

5. Vorrichtung nach Anspruch 4, wobei die Durchgangsöffnungen (5) jeweils so platziert sind, dass ihr geometrischer Mittelpunkt auf der Längsachse (4a, 4b) der länglichen Öffnung (3a, 3b) der Durchgangsöffnung am nächsten ist, oder wobei die Durchgangsöffnungen (5) von der Längsachse (4a, 4b) versetzt platziert sind.

6. Vorrichtung nach einem der vorstehenden Ansprüche, wobei die Vorrichtung eine Verbindungsachse (C) aufweist, die durch die Mitte jeder Endfläche von zwei länglichen Öffnungen (3a, 3b), die einander zugewandt sind, verläuft, oder eine Verbindungsachse (C), die durch die Mittelpunkte von mindestens zwei der in den Ansprüchen 4 und 5 definierten Vielzahl von Durchgangsöffnungen (5) verläuft, und wobei die Längsachse (4a) der ersten länglichen Öffnung (3a) einen ersten Öffnungswinkel (2a) mit der Verbindungsachse (C) bildet, und die Längsachse (4b) der zweiten länglichen Öffnung (3b) einen zweiten Öffnungswinkel (2b) mit der Verbindungsachse (C) bildet, wobei der erste Öffnungswinkel (2a) und der zweite Öffnungswinkel (2b) relativ zur Verbindungsachse (C) einander gegenüberliegend ausgebildet sind und jeweils zwischen 20 und 150 Grad, vorzugsweise jeweils zwischen 90 und 150 Grad betragen.

7. Vorrichtung (1) nach Anspruch 6, wobei der erste Öffnungswinkel (2a) und der zweite Öffnungswinkel (2b) jeweils weniger als 155 Grad, vorzugsweise weniger als 135 Grad, vorzugsweise weniger als 120 Grad betragen.

8. Vorrichtung (1) nach einem der vorstehenden Ansprüche, wobei die Längsachse der ersten länglichen Öffnung (3a) die Längsachse der zweiten länglichen Öffnung (3b) über eine Achse in der Längsebene der Vorrichtung (1) spiegelt.

9. Vorrichtung (1) nach Anspruch 8, wobei die Längsachsen der ersten länglichen Öffnung (3a) und der zweiten länglichen Öffnung (3b) in der Längsebene der Vorrichtung (1) im Wesentlichen parallel sind.

10. Vorrichtung (1) nach einem der vorstehenden Ansprüche, wobei die Form der ersten länglichen Öffnung (3a) und/oder der zweiten länglichen Öffnung (3b) stadienförmig ist.

11. Vorrichtung (1) nach einem der vorstehenden Ansprüche, wobei die Form der ersten länglichen Öffnung (3a) und/oder der zweiten länglichen Öffnung (3b) in der Längsebene der Vorrichtung (1) gekrümmt ist.

12. Vorrichtung (1) nach einem der vorstehenden Ansprüche, wobei der starre Körper entweder z-förmig, 8-förmig, sanduhrförmig, doppelt trapezförmig oder aus einer Vielzahl von Parallelogrammen gebildet ist.

13. Vorrichtung (1) nach einem der vorstehenden Ansprüche, wobei die Vorrichtung (1) in einer Kurve entlang der Länge der Vorrichtung (1) von dem ersten distalen Ende (11) zu dem zweiten distalen Ende (12) geformt ist, wobei die Kurve vorzugsweise einen Krümmungsradius zwischen 1,5 cm und 7,0 cm aufweist.

14. Vorrichtung (1) nach einem der vorstehenden Ansprüche, wobei sich die erste und die zweite längliche Öffnung (3a, 3b) mindestens teilweise parallel in der Längsrichtung der Vorrichtung (1) erstrecken, so dass sich die länglichen Öffnungen (3a, 3b) mindestens teilweise überlappen, beispielsweise um mindestens 5 %, wie beispielsweise um mindestens 15 %, wie beispielsweise um mindestens 25 % in einer seitlichen Richtung der Vorrichtung (1).

15. Kit-of-parts zur Befestigung der Vorrichtung (1) nach einem der vorstehenden Ansprüche an einem Knochen (7), umfassend:
• mindestens eine Vorrichtung (1) nach einem der vorstehenden Ansprüche;
• eine erste Schraube (8a) mit einem Durchmesser, der im Wesentlichen gleich oder kleiner als die Breite (wi) der ersten länglichen Öffnung (3a) ist,
• eine zweite Schraube (8b) mit einem Durchmesser, der im Wesentlichen gleich oder kleiner als die Breite (w₂) der zweiten länglichen Öffnung (3b) ist,
• vorzugsweise hat die erste Schraube und/oder die zweite Schraube einen Kopf, einen Gewindeteil und einen gewindelosen Teil zwischen dem Kopf und dem Gewindeteil;
das Kit zur Befestigung der Vorrichtung umfasst vorzugsweise weiter
• einen K-Draht (13);
• eine Bohrerspitze (14); und
• einen Lichtleiter (15), der eine erste Öffnung umfasst, die zur Aufnahme der Bohrerspitze konfiguriert ist, und eine zweite Öffnung, die zur Aufnahme des k-Drahtes konfiguriert ist.

## Revendications

1. Dispositif (1) de fixation à un os (7) dans une zone de plaque de croissance, ledit dispositif (1) comprenant :
• un corps rigide comprenant deux ouvertures allongées (3a, 3b), chaque ouverture allongée présentant un axe longitudinal (4a, 4b) et une largeur (w1, w2) ;
• une première partie (10) du corps rigide comprenant l'une des deux ouvertures allongées et présentant une première extrémité distale (11), et une seconde partie (20) du corps rigide comprenant l'autre des deux ouvertures allongées et présentant une seconde extrémité distale (12), distale se référant par rapport à un centre du dispositif (1), la première partie (10) et la seconde partie (20) étant raccordées l'une à l'autre en une position proximale par rapport aux extrémités distales (11, 12), la première partie (10) et la seconde partie (20) présentant l'une et l'autre une ligne centrale ;
ledit dispositif (1) étant configuré de telle façon que
• une première vis (8a) ayant essentiellement un diamètre égal ou inférieur à la largeur (wi) de la première ouverture allongée (3a) est configurée pour permettre le déplacement, au cours de la croissance longitudinale, le long de l'axe longitudinal (4a), de la première ouverture allongée (3a) lorsque ladite première vis (8a) est insérée dans la première ouverture allongée (3a), et
• une seconde vis (8b) ayant essentiellement un diamètre égal ou inférieur à la largeur (w₂) de la seconde ouverture allongée (3b) est configurée pour permettre le déplacement, au cours de la croissance longitudinale, le long de l'axe longitudinal (4b), de la seconde ouverture allongée (3b) lorsque ladite seconde vis (8a) est insérée dans la seconde ouverture allongée (3b),
**caractérisé en ce que**
• les axes longitudinaux des deux ouvertures allongées (3a, 3b) sont
∘ décalés l'un par rapport à l'autre dans une direction de la largeur des ouvertures allongées, et
∘ orientés dans des directions qui sont différentes d'au maximum 5 degrés l'une de l'autre, tels qu'étant parallèles, et
• lesdites lignes centrales étant décalées l'une par rapport à l'autre dans une direction de la largeur des ouvertures allongées (3a, 3b).

2. Dispositif (1) selon la revendication 1, comprenant en outre une partie de raccordement (30) raccordant ladite première partie (10) et ladite seconde partie (20).

3. Dispositif (1) selon la revendication 2, dans lequel la longueur de la partie de raccordement (30) est comprise entre 0,25 et 5,0 cm, telle qu'entre 0,5 cm et 2,5 cm, de préférence entre 0,5 cm et 2,0 cm.

4. Dispositif (1) selon l'une quelconque des revendications 2 et 3, dans lequel la partie de raccordement (30) comprend en outre une pluralité d'ouvertures traversantes, telles que des ouvertures circulaires (5) configurées pour fixation de broche de Kirschner.

5. Dispositif selon la revendication 4, dans lequel les ouvertures traversantes (5) sont placées chacune en ayant son centre géométrique sur l'axe longitudinal (4a, 4b) de l'ouverture allongée (3a, 3b) se trouvant le plus près de l'ouverture traversante, ou dans lequel les ouvertures traversantes (5) sont placées en étant décalées par rapport à l'axe longitudinal (4a, 4b).

6. Dispositif selon l'une quelconque des revendications précédentes, dans lequel le dispositif présente un axe de raccordement (C) passant pas le milieu de chaque face d'extrémité de deux ouvertures allongées (3a, 3b) se faisant face ou un axe de raccordement (C) passant par les centres d'au moins deux de ladite pluralité d'ouvertures traversantes (5) définies dans les revendications 4 et 5, et dans lequel l'axe longitudinal (4a) de la première ouverture allongée (3a) fait un premier angle d'ouverture (2a) avec l'axe de raccordement (C), et l'axe longitudinal (4b) de ladite seconde ouverture allongée (3b) fait un second axe d'ouverture (2b) avec l'axe de raccordement (C), ledit premier angle d'ouverture (2a) et ledit second angle d'ouverture (2b) étant formés opposés l'un à l'autre par rapport à l'axe de raccordement (C) et étant compris chacun entre 20 et 150 degrés, de préférence chacun entre 90 et 150 degrés.

7. Dispositif (1) selon la revendication 6, dans lequel ledit premier angle d'ouverture (2a) et ledit second angle d'ouverture (2b) sont chacun de moins de 155 degrés, de préférence moins de 135 degrés, de préférence moins de 120 degrés.

8. Dispositif (1) selon l'une quelconque des revendications précédentes, dans lequel l'axe longitudinal de ladite première ouverture allongée (3a) est le symétrique de l'axe longitudinal de ladite seconde ouverture allongée (3b) par rapport à un axe dans le plan longitudinal du dispositif (1).

9. Dispositif (1) selon la revendication 8, dans lequel les axes longitudinaux desdites première ouverture allongée (3a) et seconde ouverture allongée (3b) are essentiellement parallèles dans le plan longitudinal du dispositif (1).

10. Dispositif (1) selon l'une quelconque des revendications précédentes, dans lequel la forme de la première ouverture allongée (3a) et/ou de la seconde ouverture allongée (3b) est rectangulaire à petits côtés arrondis.

11. Dispositif (1) selon l'une quelconque des revendications précédentes, dans lequel la forme de la première ouverture allongée (3a) et/ou de la seconde ouverture allongée (3b) est incurvée dans le plan longitudinal du dispositif (1).

12. Dispositif (1) selon l'une quelconque des revendications précédentes, dans lequel ledit corps rigide est l'un parmi en forme de z, en forme de 8, en forme de sablier, en forme de double trapèze, ou constitué d'une pluralité de parallélogrammes.

13. Dispositif (1) selon l'une quelconque des revendications précédentes, dans lequel le dispositif (1) est formé en une courbe sur la longueur du dispositif (1) depuis la première extrémité distale (11) jusqu'à la seconde extrémité distale (12), ladite courbe ayant de préférence un rayon de courbure compris entre 1,5 cm et 7,0 cm.

14. Dispositif (1) selon l'une quelconque des revendications précédentes, dans lequel les première et seconde ouvertures allongées (3a, 3b) s'étendent au moins partiellement en parallèle dans la direction longitudinale du dispositif (1), de sorte que les ouvertures allongées (3a, 3b) se chevauchent au moins en partie, comme au moins sur 5 %, comme au moins sur 15 %, comme au moins sur 25 % dans une direction latérale du dispositif (1).

15. Trousse de pièces destinée à la fixation du dispositif (1) selon l'une quelconque des revendications précédentes à un os (7), comprenant :
• au moins un dispositif (1) selon l'une quelconque des revendications précédentes ;
• une première vis (8a) ayant un diamètre essentiellement égal ou inférieur à la largeur (w₁) de la première ouverture allongée (3a) ;
• une seconde vis (8b) ayant un diamètre essentiellement égal ou inférieur à la largeur (w₂) de la seconde ouverture allongée (3b) ;
• de préférence, la première vis et/ou la seconde vis présentent une tête, une partie filetée et une partie non filetée entre la tête et la partie filetée ;
la trousse destinée à la fixation du dispositif de préférence comprenant en outre
• une broche de Kirschner (13) ;
• un foret (14) ; et
• un guide léger (15) comprenant un premier orifice configuré pour recevoir ledit foret et un second orifice configuré pour recevoir ladite broche de Kirschner.
